# EUROPÄISCHE PATENTSCHRIFT

(11) **EP 3 723 709 B1**
(45) Veröffentlichungstag und Bekanntmachung des Hinweises auf die Patenterteilung: **31.03.2021**
(21) Anmeldenummer: 17823074.4
(22) Anmeldetag: 14.12.2017
(51) Int. Cl.: A61K 8/33, A61Q 5/02, A61Q 13/00, A61Q 15/00, A61Q 19/10, C11D 3/00

(54) **RIECHSTOFFMISCHUNGEN ENTHALTEND 8,8-DIMETHYL-6,10-DIOXASPIRO[4,5]DECAN**
PERFUME MIXTURES CONTAINING 8,8-DIMETHYL-6,10-DIOXASPIRO[4,5]DECANE
MÉLANGES DE PARFUMS COMPRENANT DU
8,8-DIMÉTHYLE-6,10-DIOXASPIRO[4,5]DÉCANE

(43) Veröffentlichungstag der Anmeldung: 21.10.2020
(73) Patentinhaber: Symrise AG, 37603 Holzminden Niedersachsen (DE)
(72) Erfinder: HÖLSCHER, Bernd, 37620 Halle (DE); MANSFELD, Marc, 37647 Brevörde (DE); AMOS, Julia, 37632 Eschershausen (DE)
(74) Vertreter: Eisenführ Speiser
(86) Internationale Anmeldenummer: PCT/EP2017/082840
(87) Internationale Veröffentlichungsnummer: WO 2019/114969

(56) Entgegenhaltungen:
- WO-A1-96/30469
- US-A- 5 711 952
- KURT KULKA: "Novel Acetals and Ketals Having the Gem Dimethyl Group", PERFUMERY AND ESSENTIAL OIL RECORD, Bd. 57, Nr. 7, 1966, Seiten 427-433, XP9503123, in der Anmeldung erwähnt
- SELL C S: "On the Unpredictability of Odor", ANGEWANDTE CHEMIE INTERNATIONAL EDITION, VERLAG CHEMIE, Bd. 45, Nr. 38, 25. September 2006 (2006-09-25), Seiten 6254-6261, XP007911257, ISSN: 1433-7851, DOI: 10.1002/ANIE.200600782 [gefunden am 2006-09-19]

## Beschreibung

Die vorliegende Erfindung betrifft primär Riechstoffmischungen, vorzugsweise Parfümöle, umfassend oder bestehend aus (a) 8,8-Dimethyl-6,10-dioxaspiro[4,5]decan, d.h. einer Verbindung der Formel (I) wie hierin beschrieben, sowie zusätzlich (b) einem oder mehreren Riechstoff(en), vorzugsweise mit einer fruchtigen Geruchsnote, ausgewählt aus der Gruppe bestehend aus Estern, Lactonen, Oximen und Schwefelverbindungen mit einer Molmasse von 240 g/mol oder weniger und/oder (c) einem oder mehreren Riechstoff(en), ausgewählt aus der Gruppe bestehend aus Acetalen, Ketonen und Ethern, mit einer Molmasse im Bereich von 126-240 g/mol. Die Erfindung betrifft zudem Verfahren zur Herstellung erfindungsgemäßer Riechstoffmischungen, Verfahren zum Verstärken der natürlichen Frische und/oder Ausstrahlung und/oder zum Maskieren oder Vermindern schwefeliger, künstlicher und/oder metallischer Noten eines oder mehrerer von der Verbindung der Formel (I) verschiedener Riechstoffe, parfümierte Produkte enthaltend eine erfindungsgemäße Riechstoffmischung, Verfahren zur Herstellung von erfindungsgemäßen parfümierten Produkten sowie die Verwendung der Verbindung der Formel (I) zum Verstärken der natürlichen Frische und/oder Ausstrahlung und/oder zum Maskierten und/oder Vermindern schwefeliger, künstlicher und/oder metallischer Noten eines oder mehrerer von der Verbindung der Formel (I) verschiedener Riechstoffe.

Weitere Aspekte und bevorzugte Ausgestaltungen der vorliegenden Erfindung ergeben sich aus den nachfolgenden Ausführungen, den beigefügten Beispielen und insbesondere den beigefügten Patentansprüchen.

Die Verbindung der Formel (I) (8,8-Dimethyl-6,10-dioxaspiro[4.5]decan, CAS Nr.: 702-75-0) 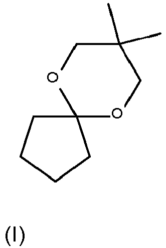 ist dem Fachmann beispielsweise aus der Veröffentlichung Perfumery and Essential Oil Record (1966), 57(7), 427-33 bekannt, worin ebenfalls eine Geruchsbeschreibung der Verbindung der Formel (I) offenbart wird. Der Geruch der Verbindung mit der Formel (I) wird dort als minzig nach Isopulegol und floral nach Geranium beschrieben. In der Parfümerie fand die Verbindung mit der Formel (I) jedoch bisher keine nennenswerte Anwendung.

Es ist des Weiteren bekannt, dass die Verbindung der Formel (I) beispielsweise durch Acetalisierung aus Cyclopentanon und 2,2-Dimethyl-1,3-propandiol hergestellt werden kann:

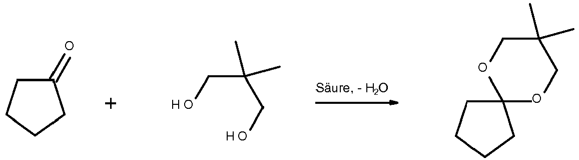

Insbesondere Fruchtriechstoffe spielen in der Parfümerie eine wichtige Rolle und sind daher in der Parfümindustrie begehrt. Es besteht zudem ein ständiger Bedarf, bestimmte geruchliche Aspekte eines Riechstoffes oder einer Riechstoffmischung zu betonen (hervorzuheben), im Falle von Fruchtriechstoffen gilt dies insbesondere für deren natürliche Frische und Ausstrahlung. Ebenso besteht ein ständiger Bedarf, bestimmte geruchliche Aspekte eines Riechstoffes oder einer Riechstoffmischung zu maskieren oder zu vermindern, im Falle von Fruchtriechstoffen gilt dies insbesondere für schwefelige, künstliche und metallische Noten.

Die primäre Aufgabe bestand daher darin, (Riech-)Stoffe bzw. (Riech-)Stoffmischungen zu finden, die die oben genannten Anforderungen erfüllen, d.h. die dazu in der Lage sind, bestimmte angenehme geruchliche Aspekte eines (insbesondere fruchtigen) Riechstoffes / einer (insbesondere fruchtigen) Riechstoffmischung zu betonen, und/oder bestimmte unangenehme geruchliche Aspekte eines (insbesondere fruchtigen) Riechstoffes / einer (insbesondere fruchtigen) Riechstoffmischung zu maskieren oder zu vermindern.

In dieser Hinsicht offenbart US 5 711 952 A die Verwendung von 7-Isopropyl-8,8-dimethyl-6,10-dioxaspiro[4.5]decan zur Veränderung des Geruchsprofils oder von Geruchsnoten anderer Riechstoffe oder Riechstoffmischungen.

Gelöst im Vergleich zum oben genannten Stand der Technik, wird diese Aufgabe überraschenderweise durch eine Riechstoffmischung, vorzugsweise ein Parfümöl, umfassend oder bestehend aus den folgenden Bestandteilen:
(a) Verbindung der Formel (I) 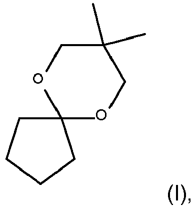 sowie zusätzlich
(b) einem oder mehreren Riechstoff(en), vorzugsweise mit einer fruchtigen Geruchsnote, ausgewählt aus der Gruppe bestehend aus Estern, Lactonen, Oximen und Schwefelverbindungen mit einer Molmasse von 240 g/mol oder weniger
   und/oder
(c) einem oder mehreren Riechstoff(en), ausgewählt aus der Gruppe bestehend aus Acetalen, Ketonen und Ethern, mit einer Molmasse im Bereich von 126-240 g/mol.

Erfindungsgemäße Riechstoffmischungen sind vorzugsweise bei 25°C und 1013 hPa flüssig und sind üblicherweise homogene Lösungen.

Die erfindungsgemäße Riechstoffmischung kann gemäß einer Ausführungsform neben dem Bestandteil (a) die Bestandteile (b) und (c) umfassen oder daraus bestehen. Gemäß einer weiteren, alternativen Ausführungsform kann die erfindungsgemäße Riechstoffmischung neben dem Bestandteil (a) entweder nur den Bestandteil (b) oder nur den Bestandteil (c) umfassen oder daraus bestehen.

Überraschenderweise bewirkt in einer erfindungsgemäßen Riechstoffmischung die Verbindung der Formel (I), dass bestimmte (angenehme) geruchliche Aspekte des oder der Riechstoffs/Riechstoffe der Komponente (b) und/oder des oder der Riechstoffs/Riechstoffe der Komponente (c) betont bzw. hervorgehoben und/oder bestimmte (unangenehme) geruchliche Aspekte derselben maskiert bzw. vermindert werden. Insbesondere schwefelige, künstliche und/oder metallische Noten der Riechstoffe der Komponenten (b) und/oder (c) werden durch die Verbindung der Formel (I) effektiv maskiert bzw. vermindert.

Überraschenderweise hat sich des Weiteren gezeigt, dass die Verbindung der Formel (I) über ihre primären sensorischen Eigenschaften hinaus zusätzliche positive Sekundäreigenschaften, wie z.B. eine hohe Stabilität unter bestimmten Anwendungsbedingungen, vorzugsweise in alkalischen Medien (Waschpulver, Wäscheweich, Seife, Shampoo etc.), aufweist.

Der Fachmann wird in einer erfindungsgemäßen Riechstoffmischung (beispielsweise einem Parfümöl) den Anteil an Komponente (a), d. h. den Anteil an der Verbindung der Formel (I), so wählen, dass der von ihm gewünschte Effekt des Betonens (Hervorhebens) und/oder Maskierens bzw. Verminderns einer Geruchsnote erreicht wird, wobei er Sorge tragen wird, keine zu große Menge der Komponente (a) einzusetzen, welche den sensorischen Gesamteindruck einer Riechstoffmischung dominieren könnte und andererseits nicht lediglich eine so geringe Menge der Komponente (a) vorzusehen, dass eine Betonung bzw. Maskierung/Verminderung geruchlicher Aspekte von Riechstoffen der Komponente (b) bzw. (c) nicht bzw. kaum noch zu spüren ist. Zu bevorzugten Konzentrationsverhältnissen siehe die nachfolgenden Ausführungen und die beigefügten Beispiele.

In erfindungsgemäßen Riechstoffmischungen wird die Verbindung der Formel (I) selbstverständlich mindestens in einer solchen Menge eingesetzt, dass eine sensorische Wirkung erzielt wird. Eine sensorische Wirkung wird durch die Anwesenheit der Verbindung der Formel (I) dann erzielt, wenn eine Vergleichs-Riechstoffmischung, die bei ansonsten identischer Zusammensetzung keine Verbindung der Formel (I) enthält, sensorisch anders bewertet wird als die erfindungsgemäße Riechstoffmischung.

Vorzugsweise wird die Verbindung der Formel (I) in einer erfindungsgemäßen Riechstoffmischung in einer solchen Konzentration eingesetzt, dass der sensorische Eindruck der erfindungsgemäßen Riechstoffmischung natürlicher, frischer, fruchtiger, mehr Ausstrahlung besitzend, weniger schwefelig, künstlich und metallisch ist als der sensorische Eindruck einer Vergleichs-Riechstoffmischung, die bei ansonsten identischer Zusammensetzung keine Verbindung der Formel (I) enthält.

Bestandteil (b) der erfindungsgemäßen Riechstoffmischung, falls vorhanden, umfasst einen oder mehrere Riechstoffe aus der Gruppe bestehend aus Estern, Lactonen, Oximen und Schwefelverbindungen mit einer Molmasse von 240 g/mol oder weniger. Bevorzugt weisen diese Riechstoffe eine fruchtige Geruchsnote auf. Solche Riechstoffe sind dem Fachmann bekannt; Ester, Lactone, Oxime und Schwefelverbindungen (auch solche mit einer fruchtigen Geruchsnote) stellen eine sehr wichtige Riechstoffgruppe in der Parfümerie dar.

Überraschenderweise werden die sensorischen Eigenschaften von Riechstoffen der Komponente (b) durch Kombination mit einer Menge der Verbindung der Formel (I) positiv beeinflusst. Im Einzelfall wird der sensorische Eindruck vorzugsweise in Richtung natürlicher, frischer, mehr Ausstrahlung, weniger schwefelig, künstlich und metallisch verschoben, wobei im Einzelfall selbstverständlich auch weitere sensorische Einflüsse zu beobachten sind. Detaillierte Geruchsbeschreibungen finden sich in den beigefügten Beispielen.

Die Riechstoffe des Bestandteils (c), falls vorhanden, fungieren regelmäßig als Fondnoten einer erfindungsgemäßen Riechstoffmischung bzw. eines erfindungsgemäßen Parfümöls. Bei den Riechstoffen des Bestandteils (c) der erfindungsgemäßen Riechstoffmischung handelt es sich um Acetale, Ketone und Ether, mit einer Molmasse im Bereich von 126-240 g/mol.

Falls bestimmte, in der erfindungsgemäßen Riechstoffmischung enthaltene Riechstoffe mehrere funktionelle Gruppen aufweisen, so dass sie ausnahmsweise gleichzeitig unter Bestandteil (b) und Bestandteil (c) fallen, so werden diese vorzugsweise - insbesondere für die Zwecke der Bestimmung der hierin beschriebenen bevorzugten Mengenangaben bzw. Massenverhältnisse - zu 50 Gew.% dem Bestandteil (b) und zu 50 Gew.% dem Bestandteil (c) zugeordnet, sofern nicht im Einzelfall anders angegeben.

Erfindungsgemäße Riechstoffmischungen, insbesondere erfindungsgemäße Parfümöle, können in flüssiger Form, unverdünnt oder mit einem Lösungsmittel verdünnt zur Parfümierung oder Aromatisierung eingesetzt werden. Geeignete Lösungsmittel hierfür sind insbesondere Ethanol, Glycerin, 1,2-Propylenglycol, 1,2-Butylenglycol, Dipropylenglycol, Diethylphthalat, Triethylcitrat, Isopropylmyristat und Triacetin.

Ether und Ester mit einer Molmasse von 240 g/mol oder weniger werden nicht zu den Bestandteilen (b) bzw. (c) gezählt, sofern es sich um eine Verbindung ausgewählt aus der Gruppe bestehend aus Dipropylenglykol, Diethylphthalat und Triacetin handelt.

Bevorzugt werden erfindungsgemäße Riechstoffmischungen, vorzugsweise erfindungsgemäße Parfümöle, mit weiteren Bestandteilen kombiniert. Bevorzugte weitere Bestandteile sind ausgewählt aus der Gruppe bestehend aus:
Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-betadicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Aromen und Geschmackstoffe sowie weitere zusätzliche Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten weiteren Riechstoffe, die nicht bereits Teil der Bestandteile (b) oder (c) einer erfindungsgemäßen Riechstoffmischung bzw. eines erfindungsgemäßen Parfümöls sind, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten. Verbindungen, die unter die Definition der Bestandteile (b) und/oder (c) fallen, werden allerdings unabhängig vom Einsatzzweck diesen Bestandteilen zugeordnet; zu Ausnahmen für bestimmte Lösungsmittel siehe oben.

Des weiteren können erfindungsgemäße Riechstoffmischungen, insbesondere erfindungsgemäße Parfümöle, an einem Trägerstoff adsorbiert sein, der sowohl für eine feine Verteilung der darin enthaltenen Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein.

Erfindungsgemäße Riechstoffmischungen, insbesondere erfindungsgemäße Parfümöle, können auch mikroverkapselt, sprühgetrocknet, als Einschluss-Komplexe oder als Extrusions-Produkte vorliegen und in dieser Form z.B. einem parfümierten Produkt (wie weiter unten beschrieben) hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der erfindungsgemäßen Riechstoffmischungen, vorzugsweise der erfindungsgemäßen Parfümöle, kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riechkompositionen können beispielsweise durch Sprühtrocknung einer die erfindungsgemäße Riechstoffmischung, vorzugsweise ein Parfümöl, enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen der erfindungsgemäßen Riechstoffmischung, vorzugsweise einem erfindungsgemäßen Parfümöl, und Cyclodextrinen oder Harnstoffderivaten in ein geeignetes Lösungsmittel, z.B. Wasser, hergestellt werden. Extrusions-Produkte können durch Verschmelzen einer erfindungsgemäßen Riechstoffmischung, vorzugsweise eines erfindungsgemäßen Parfümöls, mit einem geeigneten wachsartigen Stoff und durch Extrusion mit nachfolgender Erstarrung, ggf. in einem geeigneten Lösungsmittel, z.B. Isopropanol, erhalten werden.

Bevorzugt ist eine erfindungsgemäße Riechstoffmischung wie hierin beschrieben, wobei der Bestandteil (b) zwei, drei, vier, fünf oder mehr verschiedene Riechstoffe umfasst und/oder der Bestandteil (c) zwei, drei, vier, fünf oder mehr verschiedene Riechstoffe umfasst.

Besonders bevorzugt ist eine erfindungsgemäße Riechstoffmischung wie hierin beschrieben, wobei das Massenverhältnis der Gesamtmenge an Riechstoff(en) des Bestandteils (b), falls vorhanden, zur Verbindung der Formel (I) größer oder gleich 40 : 60, bevorzugt größer oder gleich 80 : 20, besonders bevorzugt größer oder gleich 99 : 1 , ist und/oder das Massenverhältnis der Gesamtmenge an Riechstoff(en) des Bestandteils (c), falls vorhanden, zur Verbindung der Formel (I) größer oder gleich 60 : 40 ist, bevorzugt größer oder gleich 80 : 20, besonders bevorzugt größer oder gleich 90 : 10, ist.

In eigenen Untersuchungen hat sich gezeigt, dass diese Massenverhältnisse besonders vorteilhaft sind. Bei diesen Massenverhältnissen ist der Eigengeruch der Verbindung der Formel (I) regelmäßig nicht mehr oder kaum noch wahrnehmbar, jedoch bewirkt die Anwesenheit der Verbindung der Formel (I) einen positiven Einfluss auf die Gesamtnote der erfindungsgemäßen Riechstoffmischung. Besonders überraschend ist, dass die Verbindung der Formel (I) auch in geringen Konzentrationen eine Auswirkung auf die Frische und Ausstrahlung der erfindungsgemäßen Riechstoffmischung hat, ohne in relevantem Maße selbst einen fruchtigen Geruch zu bewirken oder zu betonen.

Besonders bevorzugt ist des Weiteren eine erfindungsgemäße Riechstoffmischung wie hierin beschrieben, wobei der, ein, mehrere oder sämtliche Riechstoff(e) des Bestandteils (b), falls vorhanden, eine Molmasse im Bereich von 130 bis 240 g/mol aufweist / aufweisen.

Bevorzugt ist zudem eine erfindungsgemäße Riechstoffmischung wie hierin beschrieben, wobei der, ein, mehrere oder sämtliche Riechstoff(e) des Bestandteils (b), falls vorhanden, ausgewählt ist bzw. sind aus der Gruppe bestehend aus Allylcapronat, Allylcyclohexylpropionat, 5-Hexyl-4-methyl-tetrahydrofuran-2-on, Ethyl-2-cyclopent-2-en-1-ylacetat, 1-Cyclohexylethyl-(E/Z)-but-2-enoat, 1,5-Dimethylbicyclo[3.2.1]octan-8-on-oxim, 2,4,6-Trimethyl-4-phenyl-1,3-dioxan, 2-Methyl-4-propyl-1,3-oxathian, Isopentylacetat, Ethyl-2-(2,4-dimethyl-1,3-dioxolan-2-yl)acetat, Ethyl-2-methylbutanoat, (2-Cyclopentylcyclopentyl)-(E/Z)-but-2-enoat, 4-Methoxy-2-methyl-butan-2-thiol, 1,3-Dimethylbutyl-(E/Z)-but-2-enoat, 1,3-Dimethylbut-3-enyl-2-methylpropanoat, 1-Methoxyhexane-3-thiol, 4-Isopropyl-1-methyl-7-thiabicyclo[2.2.1]heptan, 2-(4-Methylcyclohex-3-en-1-yl)propan-2-thiol, 5-Methyl-2-(1-methyl-1-sulfanyl-ethyl)cyclohexanon, (1,3-Dimethyl-3-phenyl-butyl)acetate, Allyl-2-isopentyloxyacetat, 5-Butyltetrahydrofuran-2-on, 5-Pentyltetrahydrofuran-2-on, 5-Heptyltetrahydrofuran-2-on, 5-Octyltetrahydrofuran-2-on, 5-Hexyltetrahydrofuran-2-on, Ethyl-2-(2-methyl-1,3-dioxolan-2-yl)acetat und Diethylcyclohexan-1,4-dicarboxylat,
und/oder
der, ein, mehrere oder sämtliche Riechstoff(e) des Bestandteils (c), falls vorhanden, ausgewählt ist bzw. sind aus der Gruppe bestehend aus 1-(2,6,6-Trimethylcyclohex-2-en-1-yl)hepta-1,6-dien-3-on, 1-(2,6,6-Trimethylcyclohex-2-en-1-yl)but-2-en-1-on, 1-(2,6,6-Trimethylcyclohexen-1-yl)but-2-en-1-on, 1-(2,6,6-Trimethylcyclohex-3-en-1-yl)but-2-en-1-on, 1-(2,4,4-Trimethylcyclohex-2-en-1-yl)but-2-en-1-on, 1-Methyl-4-(2,2,3-trimethylcyclopent-3-en-1-yl)-2-oxabicyclo[2.2.2]octan, 4-(4-Methoxyphenyl)-butan-2-on, 5-Methylhept-2-en-4-on, 6,6-Dimethoxy-2,5,5-trimethyl-hex-2-en und 2,4,6-Trimethyl-4-phenyl-1,3-dioxan.

Besonders bevorzugt sind erfindungsgemäße Riechstoffmischungen, vorzugsweise erfindungsgemäße Parfümöle, wobei der, ein, mehrere oder sämtliche Riechstoffe des Bestandteils (b) ausgewählt ist bzw. sind aus der Gruppe bestehend aus Allylcapronat, Allylcyclohexylpropionate, 5-Hexyl-4-methyl-tetrahydrofuran-2-on, Ethyl-2-cyclopent-2-en-1-ylacetat, 1-Cyclohexylethyl-(E/Z)-but-2-enoate, 1,5-Dimethylbicyclo[3.2.1]octan-8-on-oxim, 2-Methyl-4-propyl-1,3-oxathian, Isopentylacetate, Ethyl-2-(2,4-dimethyl-1,3-dioxolan-2-yl)acetat, Ethyl-2-methylbutanoat, (2-Cyclopentylcyclopentyl)-(E/Z)-but-2-enoat, 4-Methoxy-2-methyl-butan-2-thiol, 1-Methoxyhexan-3-thiol, 4-lsopropyl-1-methyl-7-thiabicyclo[2.2.1]heptan, 2-(4-Methylcyclohex-3-en-1-yl)propan-2-thiol.

Ganz besonders bevorzugt sind erfindungsgemäße Riechstoffmischungen, vorzugsweise erfindungsgemäße Parfümöle, wie hierin beschrieben, in denen der Bestandteil (b) ein Ester oder eine Schwefelverbindung ist.

Weitere Beispiele für Riechstoffe, die Teil des Bestandteils (c) sein können, sind dem Fachmann bekannt und beispielsweise zu finden in S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

Ein weiterer Aspekt der vorliegenden Erfindung betrifft ein Verfahren zur Herstellung einer erfindungsgemäßen Riechstoffmischung, vorzugsweise eines Parfümöls, wie hierin beschrieben, umfassend oder bestehend aus folgendem Schritt:
- Mischen des Bestandteils (a) wie hierin definiert mit Bestandteil (b) und/oder (c) wie hierin definiert.

Bevorzugt ist ein Verfahren wie hierin beschrieben, wobei eine erfindungsgemäße Riechstoffmischung (wie hierin beschrieben), vorzugsweise ein Parfümöl, resultiert, in der
- das Massenverhältnis der Gesamtmenge an Riechstoff(en) des Bestandteils (b), falls vorhanden, zur Verbindung der Formel (I) größer oder gleich 40 : 60, bevorzugt größer oder gleich 80 : 20, besonders bevorzugt größer oder gleich 99 : 1, ist,
und/oder
- das Massenverhältnis der Gesamtmenge an Riechstoff(en) des Bestandteils (c), falls vorhanden, zur Verbindung der Formel (I) größer oder gleich 60 : 40 ist, bevorzugt größer oder gleich 80 : 20, besonders bevorzugt größer oder gleich 90 : 10, ist.

Ein weiterer Aspekt der vorliegenden Erfindung betrifft zudem ein Verfahren zum Verstärken der natürlichen Frische und/oder Ausstrahlung und/oder zum Maskieren oder Vermindern schwefeliger, künstlicher und/oder metallischer Noten eines oder mehrerer von der Verbindung der Formel (I) verschiedener Riechstoffe, vorzugsweise eines oder mehrerer von der Verbindung der Formel (I) verschiedener Riechstoffe mit einer fruchtigen Geruchsnote, umfassend den folgenden Schritt:
- Vermischen des oder der von der Verbindung der Formel (I) verschiedenen Riechstoffe mit einer Menge an Verbindung der Formel (I), die ausreicht, die natürliche Frische und/oder Ausstrahlung des oder der von der Verbindung der Formel (I) verschiedenen Riechstoffe zu verstärken und/oder schwefelige, künstliche und/oder metallische Noten des oder der von der Verbindung der Formel (I) verschiedenen Riechstoffe zu maskieren oder zu vermindern.

Besonders bevorzugt ist ein Verfahren wie hierin beschrieben, wobei der, einer, mehrere der oder sämtliche der von der Verbindung der Formel (I) verschiedene(n) Riechstoff(e) ausgewählt ist bzw. sind aus den Bestandteilen (b) und/oder (c) einer erfindungsgemäßen Riechstoffmischung (wie hierin beschrieben).

Weiter bevorzugt ist ein Verfahren wie hierin beschrieben, wobei
- das Massenverhältnis der Gesamtmenge an Riechstoff(en) des Bestandteils (b), falls vorhanden, zur Verbindung der Formel (I) größer oder gleich 40 : 60, bevorzugt größer oder gleich 80 : 20, besonders bevorzugt größer oder gleich 99 : 1 ist,
   und/oder
- das Massenverhältnis der Gesamtmenge an Riechstoff(en) des Bestandteils (c), falls vorhanden, zur Verbindung der Formel (I) größer oder gleich 60 : 40 ist, bevorzugt größer oder gleich 80 : 20, besonders bevorzugt größer oder gleich 90 : 10, ist.

Die obigen Ausführungen zu (bevorzugten) erfindungsgemäßen Riechstoffmischungen gelten für die erfindungsgemäßen Verfahren entsprechend.

Ein weiterer Aspekt der vorliegenden Erfindung betrifft ein parfümiertes Produkt enthaltend eine erfindungsgemäße Riechstoffmischung, vorzugsweise ein Parfümöl, wie hierin beschrieben, vorzugsweise in einer sensorisch wirksamen Menge.

"Sensorisch wirksame Menge" bedeutet im vorliegenden Zusammenhang, dass das erfindungsgemäße parfümierte Produkt im Betrieb bzw. bei Benutzung die sensorischen Eigenschaften der erfindungsgemäßen Riechstoffmischung erkennen lässt.

Bevorzugte erfindungsgemäße parfümierte Produkte sind ausgewählt aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

Besonders bevorzugte erfindungsgemäße parfümierte Produkte sind ausgewählt aus folgender Liste:
- Eau de Parfums, Eau de Toilettes, Rasierwässer (After-shave), Eau de Colognes, Pre-shave-Produkte, Splash-Colognes;
- saure, alkalische und neutrale Reinigungsmittel, insbesondere im Haushaltsbereich, vorzugsweise Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäscheweichspüler, Oberflächendesinfektionsmittel, insbesondere für harte Oberflächen (hard surface cleaner);
- Körperpflegemittel, vorzugsweise feste und flüssige Seifen, Duschgele, Shampoos, Rasierseifen, Rasierschäume;
- kosmetische Emulsionen vom Öl-in-Wasser-, vom Wasser-in-ÖI- und vom Wasser-in-ÖI-in-Wasser-Typ, vorzugsweise Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und - lotionen, After-shave-Cremes und -lotionen, Hautbräunungscremes und -lotionen, Hautaufhellungscremes und -lotionen;
- Haarpflegeprodukte, vorzugsweise Haarsprays, Haargele, festigende Haarlotionen, Haarspülungen, permanente und semipermanente Haarfärbemittel, Haarwässer, Haarcremes und -lotionen;
- Deodorantien und Antiperspirantien, vorzugsweise Achselsprays, Roll-ons (vorzugsweise als alkoholische oder nicht-alkoholische Lösung, als Gel oder (Mikro)Emulsion, Deosticks (Deo-Stifte), Deocremes.

Besonders bevorzugte erfindungsgemäße parfümierte Produkte sind Wasch- und Reinigungsmittel, Hygiene- oder Pflegeprodukte, insbesondere im Bereich der Körper- und Haarpflege, der Kosmetik und des Haushalts.

Bevorzugte erfindungsgemäße parfümierte Produkte sind zudem solche, bei denen der Anteil der erfindungsgemäßen Riechstoffmischung an dem parfümierten Produkt 0,01 bis 10 Gew.-%, bevorzugt 0,1 bis 5 Gew.-%, besonders bevorzugt 0,25 bis 3 Gew.-%, beträgt, jeweils bezogen auf die Gesamtmasse des parfümierten Produktes.

Die Erfindung betrifft zudem auch ein Verfahren zum Herstellen eines parfümierten Produktes, vorzugsweise eines erfindungsgemäßen parfümierten Produktes (wie hierin beschrieben), umfassend oder bestehend aus folgenden Schritten:
i) Bereitstellen einer erfindungsgemäßen Riechstoffmischung oder Herstellen einer Riechstoffmischung nach einem erfindungsgemäßen Verfahren,
ii) Bereitstellen der weiteren Bestandteile des parfümierten Produktes, und
iii) Inkontaktbringen der in Schritt ii) bereitgestellten weiteren Bestandteile des parfümierten Produkts mit einer sensorisch wirksamen Menge der in Schritt i) bereitgestellten Riechstoffmischung, vorzugsweise wobei die Menge der Verbindung der Formel (I) ausreicht, um die natürliche Frische und/oder Ausstrahlung des, eines, mehrerer oder sämtlicher Riechstoffe der Bestandteile (b) und/oder (c) zu verstärken und/oder um schwefelige, künstliche und/oder metallische Noten des, eines, mehrerer oder sämtlicher Riechstoffe der Bestandteile (b) und/oder (c) zu maskieren oder zu vermindern,
oder
I) Bereitstellen der Bestandteile des parfümierten Produktes, die keine Bestandteile (a), (b) bzw. (c) einer erfindungsgemäßen Riechstoffmischung (wie hierin beschrieben) sind,
II) Mischen der in Schritt I) bereitgestellten Bestandteile des parfümierten Produkts mit den Bestandteilen (b) und/oder (c) einer erfindungsgemäßen Riechstoffmischung, so dass eine Mischung resultiert, in welcher die Gesamtmenge der Bestandteile (b) und/oder (c) in einer sensorisch wirksamen Menge vorliegen,
III) Inkontaktbringen der in Schritt II) hergestellten Mischung mit einer Menge der Verbindung der Formel (I), vorzugsweise wobei die Menge der Verbindung der Formel (I) ausreicht, um die natürliche Frische und/oder Ausstrahlung des, eines, mehrerer oder sämtlicher Riechstoffe der Bestandteile (b) und/oder (c) zu verstärken und/oder schwefelige, künstliche und/oder metallische Noten des, eines, mehrerer oder sämtlicher Riechstoffe der Bestandteile (b) und/oder (c) zu maskieren oder zu vermindern.

Schließlich betrifft die Erfindung auch die Verwendung der Verbindung der Formel (I) 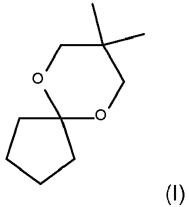 zum Verstärken der natürlichen Frische und/oder Ausstrahlung und/oder zum Maskieren oder Vermindern schwefeliger, künstlicher und/oder metallischer Noten eines oder mehrerer von der Verbindung der Formel (I) verschiedener Riechstoffe, vorzugsweise von Reichstoffen mit einer fruchtigen Geruchsnote.

Bevorzugt ist eine erfindungsgemäße Verwendung der Verbindung der Formel (I), wobei der, einer, mehrere der oder sämtliche der von der Verbindung der Formel (I) verschiedenen Riechstoffe ausgewählt ist bzw. sind aus den Bestandteilen (b) und/oder (c) einer erfindungsgemäßen Riechstoffmischung.

Für die (bevorzugten) Ausgestaltungen der hierin beschriebenen erfindungsgemäßen Verfahren, parfümierten Produkte und Verwendungen gilt das oben im Zusammenhang mit erfindungsgemäßen Riechstoffmischungen Gesagte entsprechend.

Des Weiteren gilt das im Rahmen einer hierin beschriebenen Ausführungsform der vorliegenden Erfindung Gesagte selbstverständlich jeweils auch für andere hierin beschriebene Ausführungsformen. Dementsprechend sind die hierin beschriebenen Ausführungsformen beliebig - soweit für den Fachmann sinnvoll - miteinander kombinierbar.

Die nachfolgenden Beispiele erläutern die Erfindung. Sofern nicht anders angegeben, beziehen sich Anteile und Prozente auf das Gewicht.

### Verwendete Abkürzungen:

Dipropylenglycol (DPG), Diethylphthalat (DEP), Triethylcitrat (TEC), Isopropylmyristat (IPM); nat. = natürlich;
Für Erläuterungen der Produktnamen der Riechstoffe siehe z.B. S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

### Beispiele:

### 1. Beispiel: Parfümöl P1

| | |
|---|---|
| ACETESSIGSAEUREETHYLESTER | 3 |
| ALDEHYD C14 SOG 10% DPG | 4 |
| ALLYLAMYLGLYCOLAT | 3 |
| ALLYLCYCLOHEXYLPROPIONAT | 2 |
| ALLYLHEPTYLAT 10% DPG | 3 |
| ALLYLIONON | 1,5 |
| AMBRETTOLIDE | 2 |
| AMBRINOL S 10% DPG | 1 |
| AMBROXIDE | 1 |
| BENZALDEHYD DD 10% DPG | 1 |
| BENZYLACETAT | 5 |
| BENZYLISOBUTYRAT | 0,5 |
| BENZYLSALICYLAT | 20 |
| BOURGEONAL | 1 |
| CALONE 10% DPG | 8 |
| CARYOPHYLLEN NAT. REKT. | 8 |
| CITRONELLOL 950 | 3 |
| CITRONELLYLISOBUTYRAT | 3 |
| CYCLAMENALDEHYD | 12 |
| Citrone IDENTOIL® | 30 |
| DAMASCON ALPHA 10% DPG | 3 |
| DECALACTON GAMMA | 1 |
| DIHYDROMYRCENOL | 20 |
| DIMETHYLBENZYLCARBINYLBUTYRAT | 4 |
| DIPROPYLENGLYCOL | 18,5 |
| DUPICAL 10% DPG | 1 |
| ETHYLBUTYRAT 1 % DPG | 5 |
| ETHYLMETHYLBUTYRAT-2 10% DPG | 2 |
| EVERNYL 10% DPG | 5 |
| FLORAZON | 1 |
| FLORHYDRAL 10% DPG | 3 |
| FLOROSA | 40 |
| GALAXOLIDE TYPE BASE NEW | 100 |
| GERANIOL SUPRA | 6 |
| GERANYLACETAT PUR | 5 |
| GLOBALIDE® | 10 |
| GRAPEFRUIT PHASE C | 20 |
| HEDION | 130 |
| HELIONAL | 20 |
| HELIOTROPIN/PIPERONAL 10% DPG | 2 |
| HEXENOL CIS-3 | 1 |
| HEXENYLACETAT CIS-3 10% DPG | 7 |
| HEXENYLBENZOAT CIS-3 | 1 |
| HEXENYLISOBUTYRAT CIS-3 10% DPG | 7 |
| HEXENYLSALICYLAT CIS-3 | 22 |
| HEXYLZIMTALDEHYD ALPHA | 15 |
| INDOL FF 10% DPG | 3 |
| IONON BETA | 15 |
| ISO E SUPER | 30 |
| JASMON CIS 10% DPG | 5 |
| KAROTTENSAMENOEL 10% DPG | 2 |
| LEAFOVERT® | 0,5 |
| LINALOOL | 40 |
| LINALYLACETAT | 50 |
| MACROLIDE® SUPRA | 30 |
| MAGNOLAN | 20 |
| MANDARINAL | 1 |
| MANZANATE 1 % DPG | 4 |
| METHYLANTHRANILAT 10% DPG | 1 |
| METHYLHEPTENON-6,5,2 10% DPG | 1 |
| METHYLNAPHTHYLKETON BETA 10% DPG | 1,5 |
| MINTONAT | 11 |
| ORANGENOEL | 45 |
| ORYCLON® SPEZIAL | 20 |
| PHENYLETHYLALKOHOL | 7 |
| PHENYLETHYLISOBUTYRAT | 1,5 |
| PRUNELLA | 5 |
| ROSAPHEN® | 8 |
| TETRAHYDROLINALOOL | 30 |
| VANILLIN 10% DPG | 1 |
| VERTACETAL® COEUR | 6 |

Mit Zusatz von 2% einer 10%-igen Lösung von 8,8-Dimethyl-6,10-dioxaspiro[4.5]decan (Verbindung der Formel (I)) wirkt die Mischung stärker, blumiger und grün-fruchtiger.

### 2. Beispiel: Parfümöl P2

| | |
|---|---|
| ACETESSIGSAEUREETHYLESTER | 15 |
| AMBROCENIDE® Krist. 1% DPG | 6 |
| AMBROXIDE | 10 |
| BENZYLALKOHOL DD | 18 |
| BERGAMOTTOEL BERGAPTENFREI | 40 |
| CARDAMOMENOEL 10% DPG | 10 |
| CEDERNHOLZOEL | 10 |
| CEDRAMBER | 40 |
| CITRONENOEL ITAL. | 38 |
| CUMARIN 10% DPG | 5 |
| DAMASCENON 10% DPG | 5 |
| DAVANAOEL F. PARF. 10% DPG | 7,5 |
| DIMETHYLBENZYLCARBINYLACETAT 10% DPG | 4,5 |
| DIPROPYLENGLYCOL | 52 |
| ETHYLHEPTYLAT 10% DPG | 1,5 |
| ETHYLPROPIONAT 10% DPG | 4,5 |
| EVERNYL | 2 |
| FLOROSA | 10 |
| GERANIOL SUPRA | 5 |
| GLOBALIDE® | 10 |
| GRAPEFRUITOEL | 20 |
| HEDION HC/30 | 50 |
| INGWEROEL 10% DPG | 5 |
| IONON BETA | 10 |
| ISO E SUPER | 350 |
| JASMAPRUNAT 10% DPG | 10,5 |
| KRAUSEMINZOEL 65% AMERIK. | 10 |
| LIMETTENOEL KALTGEPRESST | 20 |
| LINALOOL | 40 |
| LINALYLACETAT | 20 |
| LORBEERBLAETTEROEL FLS | 5 |
| MAJANTOL® | 30 |
| MANDARINENOEL ITAL. | 2 |
| NEROLI ARTESSENCE | 4 |
| ORANGENBLUETEN ABS. 10% DPG | 2 |
| ORANGENOEL BITTER | 6 |
| ORANGENOEL ITAL.SUESS ENTF. | 10 |
| PATCHOULIOEL ENTF. DM | 15 |
| PERUBALSAMOEL ED | 1,5 |
| PETITGRAINOEL PARAG. BOLEADOR | 2 |
| ROSMARINOEL TUN. | 5 |
| SANDELHOLZOEL OSTINDISCH | 2 |
| SANDRANOL | 2 |
| SULTANENE® 1% DPG | 1,5 |
| TABANON COEUR | 3 |
| THYMIANOEL WEISS 1% DPG | 10 |
| TOLU RESIN 10% DPG | 7,5 |
| VERTOFIX | 50 |
| VETIVEROEL HAITI | 5 |
| VERTACTAL COEUR | 2 |
| ZIMTRINDENOEL MADAG. 10% DPG | 5 |

Mit Zusatz von 3% 8,8-Dimethyl-6,10-dioxaspiro[4.5]decan (Verbindung der Formel (I)) wird die Mischung frischer, die Fruchtnoten werden verstärkt und die Verbindung der Formel (I) sorgt für eine harmonische, natürliche Note.

### 3. Beispiel: Parfümöl P3

| | |
|---|---|
| ALDEHYD C 8 | 3,5 |
| ALDEHYD C10 | 6,5 |
| GLYCOLIERRAL | 10 |
| FLOROPAL | 45 |
| RHUBAFURANE | 2,5 |
| STYROLYLACETAT | 25 |
| ORANGENOEL | 304 |
| GRAPEFRUITOEL | 50 |
| GRAPEFRUITOEL TERPENE | 250 |
| AMAROCIT® | 45 |
| CORPS 1490 10% DPG | 3,5 |
| ALLYLHEPTYLAT | 3,5 |
| NECTARYL | 5 |
| THIOMENTHANON-8,3 10% DPG | 6,5 |
| LINALOOL | 95 |
| NEROL 900 | 35 |
| HEDION | 100 |

Mit Zusatz von 5% 8,8-Dimethyl-6,10-dioxaspiro[4.5]decan (Verbindung der Formel (I)) wird die Mischung natürlicher, weniger schwefelig, fruchtiger, runder, frischer und spritziger.

### 4. Beispiel: Parfümöl P4

| | |
|---|---|
| HEXENOL CIS-3 | 7,5 |
| STEMONE | 10 |
| ORANGENOEL | 75 |
| CORPS 1490 1% DPG | 6 |
| OXANE 10% DPG | 3,5 |
| PINEN BETA NAT. | 120 |
| THIOMENTHANON-8,3 10% DPG | 3,5 |
| LINALOOL | 95 |
| LINALOOLOXID | 3,5 |
| DIMETHYLBENZYLCARBINYLACETAT | 15 |
| ROSENOXID HIGH CIS | 5 |
| PHENYLETHYLALKOHOL | 95 |
| CITRONELLOL 950 | 120 |
| HEDION | 90 |
| VELOUTONE | 6 |
| ANISYLACETAT | 20 |
| ZIMTALKOHOL | 5 |
| DIPROPYLENGLYCOL | 310 |

Mit Zusatz von 10% 8,8-Dimethyl-6,10-dioxaspiro[4.5]decan (Verbindung der Formel (I)) wird die Mischung weniger schwefelig, stärker fruchtiger, runder, blumiger und natürlicher.

### 5. Beispiel: Geruchsbeschreibung von bevorzugten Riechstoffen nach Zusatz von 8,8-Dimethyl-6,10-dioxaspiro[4.5]decan (Verbindung der Formel (I))

| **Riechstoffe** | **Typ** | **Dosierung Verbindung der Formel (I)** | **Geruchsbeschreibung im Vergleich zum Geruch des reinen Riechstoffs** |
|---|---|---|---|
| Sultanene (M=154) | Ester | 1% | mehr Impact, frischer, natürlicher, stärker fruchtiger, strahlender |
| Aprifloren (M=184) | Lacton | 0,2% | stärker fruchtiger, saftiger |
| Datilat (M=196) | Ester | 0,4% | mehr Impact und Strahlung, fruchtiger, spritziger |
| Buccoxim (M=167) | Oxim | 0,2% | weniger schwefelig, stärker, fruchtiger, natürlicher, mehr Volumen |
| Vertacetal Coeur (M=206) | Acetal | 0,2% | weniger metallisch, runder, natürlich, stärker fruchtiger |
| Oxanthia 50% in TEC (M=160) | Schwefelverbindung | 0,7% | weniger schwefelig, stärker fruchtiger, natürlicher |
| Cassiffix (M=234) | Ether | 0,3% | mehr Impact, saftigerfruchtiger |
| Isoamylacetat (M=130) | Ester | 0,7% | mehr Banane, saftigerfrischer |
| Ethyl Methyl Butyrat-2 (M=130) | Ester | 0,5% | weniger metallisch, fruchtiger, runder |
| Fragolane (M=188) | Ester/Acetal | 0,3% | mehr Erdbeere, frischer, natürlicher |
| Pyroprunat (M=222) | Ester | 0,5% | fruchtig-süßer, stärker nach reifer Frucht |
| Cassix 150 (M=134) | Schwefelverbindung | 1% | weniger Schwefelnote, weicher, runder und mehr Natürlichkeit |
| Allycyclohexylpropionat (M=196) | Ester | 0,6% | mehr Impact, frischer und strahlender |
| Allycapronat (M=156) | Ester | 0,6% | mehr Impact, wässrigertransparenter, frischer |
| Frutinat (M=170) | Ester | 0,7% | mehr Impact, saftiger und fruchtiger |
| Isopentyrate (M=170) | Ester | 0,6% | mehr Impact, strahlender, frischer |
| Buccoflor (M=148) | Schwefelverbindung | 0,5% | mehr Impact, saftiger, frischer und fruchtiger |
| Thiocineol (M=170) | Schwefelverbindung | 1% | weniger Schwefel- und Zwiebelnote, mehr fruchtiger und saftiger |
| Thiomenthanone-8,8 (M=148) | Schwefelverbindung | 1% | weniger Schwefelnote, mehr fruchtiger und saftiger |

### Herstellvorschrift des 8.8-Dimethyl-6,10-dioxaspiro[4.5]decans (Verbindung der Formel (I))

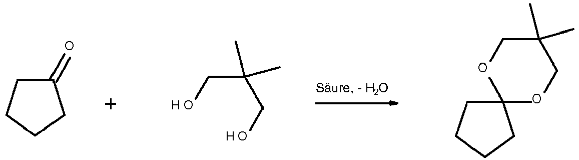

In einem 1000 ml-Dreihalskolben mit Rührer, Kontaktthermometer, Pilzheizhaube, und Wasserabscheider, werden 126 g (1.5 mol) Cyclopentanon, 156 g (1.5 mol) 2,2-Dimethyl-1,3-propandiol, 1,5 g p-Toluolsulfonsäure in 305 g Cyclohexan vorgelegt und 2-3 am Wasserabscheider gesiedet. Nach Abkühlen auf RT wird 2x mit Soda-Lsg. gewaschen und eingeengt. Der Rückstand wird an der 10 cm M.F.K. destilliert (Siedebereich: 72-73°C/10mbar).
Ausbeute: 219 g (entspricht 85,9% d. Th.)
MS: m/z (%) = 170 (7), 141(100), 85(34), 69(68), 67(13), 56(43), 55(67), 41(33).
¹H-NMR (400 MHz, DMSO-d6) δ 3.39 (s, 4H), 1.82 - 1.73 (m, 4H), 1.62 - 1.50 (m, 4H), 0.89 (s, 6H).

### Formulierungsbeispiele

Die Parfumöle P1, P2, P3 bzw. P4 aus den obigen Parfümöl-Beispielen 1 bis 4 wurden jeweils separat in die nachfolgenden Formulierungen eingearbeitet.

Die oben unter dem jeweiligen Parfümöl beschriebenen geruchlichen Effekte wurden jeweils auch in den nachfolgenden Formulierungen beobachtet.

### Beispiel F1 - Waschpulver

| **Material** | **Chemischer Name** | **Funktion** | **Gew.-%** | **Gew.-%** |
|---|---|---|---|---|
| Natrium Metasilicat Pentahydrat | Sodium Metasilicate Pentahydrate | | Ad 100 | Ad 100 |
| Natriumhydrogencarbonat | Sodium hydrogen carbonate | Alkali | 15,0 | 15,0 |
| Natriumpercarbonat | Sodium carbonate peroxyhydrate | Bleichmittel | 15,0 | 15,0 |
| Peractive AC Blue | TAED / Na-Carboxymethylcellulose | Aktivator | 5,00 | 5,00 |
| Genapol OA-080 | Oxoalkohol C14-15, 8EO | Nichtionisches Tensid | 3,00 | 3,00 |
| Texapon K12 Pulver | Sodium Lauryl Sulphate C12 | Anionisches Tensid | 7,00 | 7,00 |
| Tinopal CBS-X | | Aufheller | 0,50 | 0,50 |
| Savinase 6.0 T, Type W | Protease | Enzym | 0,40 | 0,40 |
| Termamyl 120 T | Alpha-Amylase | Enzym | 0,30 | 0,30 |
| Natriumsulfat | Sodium Sulphate | Füllstoff | 5,50 | 5,50 |
| Parfumöl P1, P2, P3 bzw. P4 | | Parfum (Fragrance) | 0,30 | 0,50 |

### Beispiel F2 - Allzweckreiniger

| **Material** | **Chemischer Name** | **Funktion** | **Gew.-%** | **Gew.-%** |
|---|---|---|---|---|
| Entionisiertes Wasser | Water | Lösungsmittel | Ad 100 | Ad 100 |
| Mergal K9N | 5-Chloro-2-methyl-3-(2H)-isothiazolone und 2-methyl-3-(2H)-isothiazolone | Konservierungsmittel | 0,1 | 0,1 |
| Trinatriumcitrat Dihydrat | Tri Sodium Citrate Dihydrate | Komplexbildner | 3,0 | 3,0 |
| Zetesol NL-2 | Fatty alcohol C12-14-sulfate, Sodium | Anionisches Tensid | 30,0 | 30,0 |
| Imbentin C/125/055 | Fatty alcohol C12-C15, 8EO | Nichtionisches Tensid | 5,0 | 5,0 |
| Ethanol | Ethanol | Lösungsmittel | 2,0 | 2,0 |
| Parfumöl P1, P2, P3 bzw. P4 | | Parfum (Fragrance) | 0,3 | 0,5 |

### Beispiel F3 - Shampoo

| **Material** | **INCI-Name** | **Gew.-%** | **Gew.-%** |
|---|---|---|---|
| Entionisiertes Wasser | Water | Ad 100 | Ad 100 |
| Plantacare PS 10 | Sodium Laureth Sulfate, Lauryl Glucoside | 20,0 | 20,0 |
| Euperlan PK 771 | Glycol Distearate, Sodium Lauryl Sulfate, Cocamide MEA, Lau reth-10 | 6,0 | 6,0 |
| Dragocid Liquid | Phenoxyethanol, Methylparaben, Ethylparaben, Butylparaben, Propylparaben, Isobutylparaben | 0,5 | 0,5 |
| Natrium Chlorid | Sodium Chloride | 1,4 | 1,4 |
| Citronensäure Monohydrat kristallin | Citric Acid | 0,1 | 0,1 |
| Parfumöl P1, P2, P3 bzw. P4 | Parfum (Fragrance) | 0,5 | 0,8 |

### Beispiel F4 - Duschgel

| **Material** | **INCI-Name** | **Gew.-%** | **Gew.-%** |
|---|---|---|---|
| Entionisiertes Wasser | Wasser | Ad 100 | Ad 100 |
| Plantacare PS 10 | Sodium Laureth Sulfate, Lauryl Glucoside | 20,0 | 20,0 |
| Dragocid Liquid | Phenoxyethanol, Methylparaben, Ethylparaben, Butylparaben, Propylparaben, Isobutylparaben | 0,5 | 0,5 |
| Natriumchlorid | Sodium Chloride | 1,4 | 1,4 |
| Citronensäure Monohydrat kristallin | Citric Acid | 1,3 | 1,3 |
| Parfumöl P1, P2, P3 bzw. P4 | Parfum (Fragrance) | 0,5 | 0,7 |

### Beispiel F5 - Weichspüler

| **Material** | **Chemischer Name** | **Funktion** | **Gew.-%** | **Gew.-%** |
|---|---|---|---|---|
| Entionisiertes Wasser | Wasser | Lösungsmittel | Ad 100 | Ad 100 |
| Rewoquat WE 18 | Dialkylesterammoniumethosulfate | Kationisches Tensid | 16,6 | 16,6 |
| Mergal K9N | 5-Chloro-2-methyl-3-(2H)-isothiazolone und 2-methyl-3-(2H)-isothiazolone | Konservierungsmittel | 0,10 | 0,10 |
| Dow Corning 1520 Antifoam | Polydimethyl-siloxane | Entschäumer | 0,30 | 0,30 |
| Magnesium Chlorid 1%ige Lösung | Magnesium Chlorid Lösung | Konsistenzgeber | 10,00 | 10,00 |
| Parfumöl P1, P2, P3 bzw. P4 | | Fragrance | 0,55 | 0,75 |

### Beispiel F6 - Eau de Cologne / Eau de Toilette

| **Inhaltsstoffe** | **Gew.-%** | **Gew.-%** |
|---|---|---|
| Parfumöl P1, P2, P3 bzw. P4 | 5 | 10 |
| Ethanol | Ad 100 | Ad 100 |
| Wasser | 18 | 10 |

### Beispiel F7 - Aerosol-Pumpsprav

| **Inhaltsstoffe** | **Gew.-%** | **Gew.-%** |
|---|---|---|
| Parfumöl P1, P2, P3 bzw. P4 | 1,0 | 1,5 |
| Ethanol | Ad 100 | Ad 100 |
| Wasser | 5,0 | 8,0 |
| Alpha- Tocopherol | 0,20 | 0,20 |
| Hydroxypropylcellulose | 0,20 | - |
| Rosmarinextrakt, in Ethanol löslich | 0,22 | - |
| Cetylalkohol | 1,00 | 0,50 |

### Beispiel F8 - Shampoo

| **Inhaltsstoffe** | **Gew.-%** | **Gew.-%** | **Gew.-%** |
|---|---|---|---|
| Natriumlaurylethersulfat (z.B. Texapon NSO, Fa. Cognis Deutschland GmbH) | 12 | 12 | 12 |
| Cocamidopropylbetain (z.B. Dehyton K, Fa. Cognis Deutschland GmbH) | 2 | 2 | 2 |
| Natriumchlorid | 1,4 | 1,4 | 1,4 |
| Citronensäure | 1,3 | 1,3 | 1,3 |
| Phenoxyethanol, Methyl-, Ethyl-, Butyl- und Propylparaben | 0,5 | 0,5 | 0,5 |
| Parfumöl P1, P2, P3 bzw. P4 | 0,3 | 0,5 | 0,7 |
| Wasser | Ad 100 | Ad 100 | Ad 100 |

### Beispiel F9 - Waschpulver

| **Inhaltsstoffe** | **Gew.-%** | **Gew.-%** | **Gew.-%** |
|---|---|---|---|
| Lineares Na-Alkylbenzolsulfonat | 8,8 | 8,8 | 8,8 |
| Ethoxylierter Fettalkohol C12-18 (7 EO) | 4,7 | 4,7 | 4,7 |
| Na-Seife | 3,2 | 3,2 | 3,2 |
| Entschäumer DOW CORNING(R) 2-4248S POWDERED ANTIFOAM, Silikonöl auf Zeolith als Trägermaterial | 3,9 | 3,9 | 3,9 |
| Zeolith 4A | Ad 100 | Ad 100 | Ad 100 |
| Na-Carbonat | 11,6 | 11,6 | 11,6 |
| Na-Salz eines Copolymers aus Acryl- und Maleinsäure (Sokalan CP5) | 2,4 | 2,4 | 2,4 |
| Na-Silicat | 3,0 | 3,0 | 3,0 |
| Carboxymethylcellulose | 1,2 | 1,2 | 1,2 |
| Dequest 2066([[(Phosphonomethyl)imino]bis[(ethylennitrilo)bis (methylen)]]tetrakis-phosphonsäure, Natriumsalz) | 2,8 | 2,8 | 2,8 |
| Optischer Aufheller | 0,2 | 0,2 | 0,2 |
| Na-Sulfat | 6,5 | 6,5 | 6,5 |
| Protease | 0,4 | 0,4 | 0,4 |
| Natriumperborat tetrahydrat | 21,7 | 21,7 | 21,7 |
| Parfumöl P1, P2, P3 bzw. P4 | 0,25 | 0,35 | 0,5 |
| EDTA | 1,0 | 1,0 | 1,0 |

### Beispiel F10 - Flüssigwaschmittel

| **Inhaltsstoffe** | **Gew.-%** |
|---|---|
| Entionisiertes Wasser | 39,9 |
| Optischer Aufheller | 0,10 |
| Kokos Fettsäuren (C12-C18) | 7,5 |
| Kaliumhydroxid 50%ige Lösung | 4,3 |
| Propan-1,2-diol | 5,00 |
| Fettalkohole C12-C15, 8 EO | 12,0 |
| Na-Salz sekundärer Alkylsulfonate (C13-C17) | 17,0 |
| Triethanolamin | 2,0 |
| Trinatriumcitrat Dihydrat | 5,0 |
| Dequest 2066([[(Phosphonomethyl)imino]bis[(ethylennitrilo)bis (methylen)]]tetrakis-phosphonsäure, Natriumsalz) | 3,0 |
| Ethanol | 3,0 |
| Enzyme | 0,7 |
| Parfumöl P1, P2, P3 bzw. P4 | 0,5 |

### Beispiel F11 - Flüssigwaschmittel Konzentrat

| **Inhaltsstoffe** | **Gew.-%** |
|---|---|
| Entionisiertes Wasser | 13,4 |
| Kokos Fettsäuren (C12-C18) | 10,0 |
| Fettalkohole C12-C15, 8 EO | 26,0 |
| Na-Salz sekundärer Alkylsulfonate (C13-C17) | 26,5 |
| Triethanolamin | 8,5 |
| Na-Salz von Fettalkoholsulfaten C12-C14 | 3,0 |
| Ethanol | 5,5 |
| Harnstoff | 4,5 |
| Enzyme | 0,9 |
| Citronensäure | 1,0 |
| Parfumöl P1, P2, P3 bzw. P4 | 0,7 |

## Patentansprüche

1. Riechstoffmischung, vorzugsweise Parfümöl, umfassend oder bestehend aus den folgenden Bestandteilen:
(a) Verbindung der Formel (I) 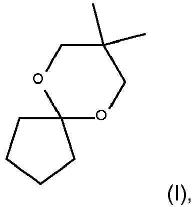 sowie zusätzlich
(b) einem oder mehreren Riechstoff(en), vorzugsweise mit einer fruchtigen Geruchsnote, ausgewählt aus der Gruppe bestehend aus Estern, Lactonen, Oximen und Schwefelverbindungen mit einer Molmasse von 240 g/mol oder weniger
und/oder
(c) einem oder mehreren Riechstoff(en), ausgewählt aus der Gruppe bestehend aus Acetalen, Ketonen und Ethern, mit einer Molmasse im Bereich von 126-240 g/mol.

2. Riechstoffmischung nach Anspruch 1, wobei der Bestandteil (b) zwei, drei, vier, fünf oder mehr verschiedene Riechstoffe umfasst
und/oder
der Bestandteil (c) zwei, drei, vier, fünf oder mehr verschiedene Riechstoffe umfasst.

3. Riechstoffmischung nach Anspruch 1 oder 2, wobei das Massenverhältnis der Gesamtmenge an Riechstoff(en) des Bestandteils (b), falls vorhanden, zur Verbindung der Formel (I) größer oder gleich 40 : 60, bevorzugt größer oder gleich 80 : 20, besonders bevorzugt größer oder gleich 99 : 1 , ist
und/oder
das Massenverhältnis der Gesamtmenge an Riechstoff(en) des Bestandteils (c), falls vorhanden, zur Verbindung der Formel (I) größer oder gleich 60 : 40 ist, bevorzugt größer oder gleich 80 : 20, besonders bevorzugt größer oder gleich 90 : 10, ist.

4. Riechstoffmischung nach einem der vorangehenden Ansprüche, wobei der, ein, mehrere oder sämtliche Riechstoff(e) des Bestandteils (b), falls vorhanden, eine Molmasse im Bereich von 130 bis 240 g/mol aufweist/aufweisen.

5. Riechstoffmischung nach einem der vorangehenden Ansprüche, wobei der, ein, mehrere oder sämtliche Riechstoff(e) des Bestandteils (b), falls vorhanden, ausgewählt ist bzw. sind aus der Gruppe bestehend aus Allylcapronat, Allylcyclohexylpropionat, 5-Hexyl-4-methyl-tetrahydrofuran-2-on, Ethyl-2-cyclopent-2-en-1-ylacetat, 1-Cyclohexylethyl-(E/Z)-but-2-enoat, 1,5-Dimethylbicyclo[3.2.1]octan-8-on oxim, 2,4,6-Trimethyl-4-phenyl-1,3-dioxan, 2-Methyl-4-propyl-1,3-oxathian, Isopentylacetat, Ethyl-2-(2,4-dimethyl-1,3-dioxolan-2-yl)acetat, Ethyl-2-methylbutanoat, (2-Cyclopentylcyclopentyl)-(E/Z)-but-2-enoat, 4-Methoxy-2-methyl-butan-2-thiol, 1,3-Dimethylbutyl-(E/Z)-but-2-enoat, 1,3-Dimethylbut-3-enyl-2-methylpropanoat, 1-Methoxyhexane-3-thiol, 4-lsopropyl-1-methyl-7-thiabicyclo[2.2.1]heptan, 2-(4-Methylcyclohex-3-en-1-yl)propan-2-thiol, 5-Methyl-2-(1-methyl-1-sulfanyl-ethyl)cyclohexanon, (1,3-Dimethyl-3-phenylbutyl)acetate, Allyl-2-isopentyloxyacetat, 5-Butyltetrahydrofuran-2-on, 5-Pentyltetrahydrofuran-2-on, 5-Heptyltetrahydrofuran-2-on, 5-Octyltetrahydrofuran-2-on, 5-Hexyltetrahydrofuran-2-on, Ethyl-2-(2-methyl-1,3-dioxolan-2-yl)acetat und Diethylcyclohexan-1,4-dicarboxylat,
und/oder
der, ein, mehrere oder sämtliche Riechstoff(e) des Bestandteils (c), falls vorhanden, ausgewählt ist bzw. sind aus der Gruppe bestehend aus 1-(2,6,6-Trimethylcyclohex-2-en-1-yl)hepta-1,6-dien-3-on, 1-(2,6,6-Trimethylcyclohex-2-en-1-yl)but-2-en-1-on, 1-(2,6,6-Trimethylcyclohexen-1-yl)but-2-en-1-on, 1-(2,6,6-Trimethylcyclohex-3-en-1-yl)but-2-en-1-on, 1-(2,4,4-Trimethylcyclohex-2-en-1-yl)but-2-en-1-on, 1-Methyl-4-(2,2,3-trimethylcyclopent-3-en-1-yl)-2-oxabicyclo[2.2.2]octan, 4-(4-Methoxyphenyl)-butan-2-on, 5-Methylhept-2-en-4-on, 6,6-Dimethoxy-2,5,5-trimethyl-hex-2-en und 2,4,6-Trimethyl-4-phenyl-1,3-dioxan.

6. Verfahren zur Herstellung einer Riechstoffmischung, vorzugsweise eines Parfümöls, nach einem der Ansprüche 1 bis 5, umfassend oder bestehend aus folgendem Schritt:
- Mischen des Bestandteils (a) wie in einem der Ansprüche 1 bis 5 definiert mit Bestandteil (b) und/oder (c) wie in einem der Ansprüche 1 bis 5 definiert.

7. Verfahren nach Anspruch 6, wobei eine Riechstoffmischung, vorzugsweise ein Parfümöl, resultiert, in der
- das Massenverhältnis der Gesamtmenge an Riechstoff(en) des Bestandteils (b), falls vorhanden, zur Verbindung der Formel (I) größer oder gleich 40 : 60, bevorzugt größer oder gleich 80 : 20, besonders bevorzugt größer oder gleich 99 : 1, ist,
und/oder
- das Massenverhältnis der Gesamtmenge an Riechstoff(en) des Bestandteils (c), falls vorhanden, zur Verbindung der Formel (I) größer oder gleich 60 : 40 ist, bevorzugt größer oder gleich 80 : 20, besonders bevorzugt größer oder gleich 90 : 10, ist.

8. Verfahren zum Verstärken der natürlichen Frische und/oder Ausstrahlung und/oder zum Maskieren oder Vermindern schwefeliger, künstlicher und/oder metallischer Noten eines oder mehrerer von der Verbindung der Formel (I) verschiedener Riechstoffe, vorzugsweise eines oder mehrerer von der Verbindung der Formel (I) verschiedener Riechstoffe mit einer fruchtigen Geruchsnote, umfassend den folgenden Schritt:
- Vermischen des oder der von der Verbindung der Formel (I) verschiedenen Riechstoffe mit einer Menge an Verbindung der Formel (I), die ausreicht, die natürliche Frische und/oder Ausstrahlung des oder der von der Verbindung der Formel (I) verschiedenen Riechstoffe zu verstärken und/oder schwefelige, künstliche und/oder metallische Noten des oder der von der Verbindung der Formel (I) verschiedenen Riechstoffe zu maskieren oder zu vermindern.

9. Verfahren nach Anspruch 8, wobei der, einer, mehrere der oder sämtliche der von der Verbindung der Formel (I) verschiedene(n) Riechstoff(e) ausgewählt ist bzw. sind aus den Bestandteilen (b) und/oder (c) einer Riechstoffmischung nach einem der Ansprüche 1 bis 5.

10. Verfahren nach Anspruch 9, wobei
- das Massenverhältnis der Gesamtmenge an Riechstoff(en) des Bestandteils (b), falls vorhanden, zur Verbindung der Formel (I) größer oder gleich 40 : 60, bevorzugt größer oder gleich 80 : 20, besonders bevorzugt größer oder gleich 99 : 1 ist,
und/oder
- das Massenverhältnis der Gesamtmenge an Riechstoff(en) des Bestandteils (c), falls vorhanden, zur Verbindung der Formel (I) größer oder gleich 60 : 40 ist, bevorzugt größer oder gleich 80 : 20, besonders bevorzugt größer oder gleich 90 : 10, ist.

11. Parfümiertes Produkt enthaltend eine Riechstoffmischung, vorzugsweise ein Parfümöl, nach einem der Ansprüche 1 bis 5, vorzugsweise in einer sensorisch wirksamen Menge.

12. Parfümiertes Produkt nach Anspruch 11, wobei das Produkt ausgewählt ist aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und - lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe
und/oder
wobei der Anteil der Riechstoffmischung nach einem der Ansprüche 1 bis 5 an dem parfümierten Produkt 0,01 bis 10 Gew.-%, bevorzugt 0,1 bis 5 Gew.-%, besonders bevorzugt 0,25 bis 3 Gew.-%, beträgt, jeweils bezogen auf die Gesamtmasse des parfümierten Produktes.

13. Verfahren zum Herstellen eines parfümierten Produktes, vorzugsweise eines Produktes nach Anspruch 11 oder 12, umfassend oder bestehend aus folgenden Schritten:
i) Bereitstellen einer Riechstoffmischung nach einem der Ansprüche 1 bis 5 oder Herstellen einer Riechstoffmischung nach einem Verfahren nach einem der Ansprüche 6 oder 7,
ii) Bereitstellen der weiteren Bestandteile des parfümierten Produktes, und
iii) Inkontaktbringen der in Schritt ii) bereitgestellten weiteren Bestandteile des parfümierten Produkts mit einer sensorisch wirksamen Menge der in Schritt i) bereitgestellten Riechstoffmischung, vorzugsweise wobei die Menge der Verbindung der Formel (I) ausreicht, um die natürliche Frische und/oder Ausstrahlung des, eines, mehrerer oder sämtlicher Riechstoffe der Bestandteile (b) und/oder (c) zu verstärken und/oder um schwefelige, künstliche und/oder metallische Noten des, eines, mehrerer oder sämtlicher Riechstoffe der Bestandteile (b) und/oder (c) zu maskieren oder zu vermindern,
oder
I) Bereitstellen der Bestandteile des parfümierten Produktes, die keine Bestandteile (a), (b) bzw. (c) einer Riechstoffmischung nach einem der Ansprüche 1 bis 5 sind,
II) Mischen der in Schritt I) bereitgestellten Bestandteile des parfümierten Produkts mit den Bestandteilen (b) und/oder (c) einer Riechstoffmischung nach einem der Ansprüche 1 bis 5, so dass eine Mischung resultiert, in welcher die Gesamtmenge der Bestandteile (b) und/oder (c) in einer sensorisch wirksamen Menge vorliegen,
III) Inkontaktbringen der in Schritt II) hergestellten Mischung mit einer Menge der Verbindung der Formel (I), vorzugsweise wobei die Menge der Verbindung der Formel (I) ausreicht, um die natürliche Frische und/oder Ausstrahlung des, eines, mehrerer oder sämtlicher Riechstoffe der Bestandteile (b) und/oder (c) zu verstärken und/oder schwefelige, künstliche und/oder metallische Noten des, eines, mehrerer oder sämtlicher Riechstoffe der Bestandteile (b) und/oder (c) zu maskieren oder zu vermindern.

14. Verwendung der Verbindung der Formel (I) 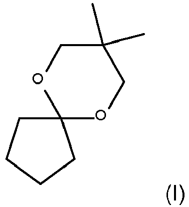 zum Verstärken der natürlichen Frische und/oder Ausstrahlung und/oder zum Maskieren oder Vermindern schwefeliger, künstlicher und/oder metallischer Noten eines oder mehrerer von der Verbindung der Formel (I) verschiedener Riechstoffe, vorzugsweise von Reichstoffen mit einer fruchtigen Geruchsnote,
wobei vorzugsweise der, einer, mehrere der oder sämtliche der von der Verbindung der Formel (I) verschiedenen Riechstoffe ausgewählt ist bzw. sind aus den Bestandteilen (b) und/oder (c) einer Riechstoffmischung nach einem der Ansprüche 1 bis 5.

## Claims

1. Fragrance substance mixture, preferably perfume oil, comprising or consisting of the following components:
(a) Compound of formula (I) 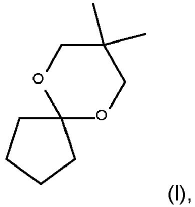 and additionally
(b) one or more fragrance substance(s), preferably with a fruity olfactory note, selected from the group consisting of esters, lactones, oximes and sulphur compounds with a molecular weight of 240 g/mol or less
and/or
(c) one or more fragrance substance(s), selected from the group consisting of acetals, ketones and ethers, with a molecular weight in the range of 126-240 g/mol.

2. Fragrance substance mixture according to claim 1, wherein component (b) comprises two, three, four, five or more different fragrance substances
and/or
component (c) comprises two, three, four, five or more different fragrance substances.

3. Fragrance substance mixture according to claim 1 or 2, wherein the weight ratio of the total amount of fragrance substance(s) of component (b), if present, to the compound of formula (I) is greater than or equal to 40:60, preferably greater than or equal to 80:20, particularly preferably greater than or equal to 99:1,
and/or
the weight ratio of the total amount of fragrance substance(s) of component (c), if present, to the compound of formula (I) is greater than or equal to 60 : 40, preferably greater than or equal to 80 : 20, particularly preferably greater than or equal to 90 : 10.

4. Fragrance substance mixture according to any of the preceding claims, wherein the, one, several or all of the fragrance substance(s) of component (b), if present, has/have a molecular weight in the range of 130 to 240 g/mol.

5. Fragrance substance mixture according to any of the preceding claims, wherein the, one, several or all of the fragrance substance(s) of component (b), if present, is or are selected from the group consisting of allyl capronate, allyl cyclohexyl propionate, 5-hexyl-4-methyl-tetrahydrofuran-2-one, ethyl 2-cyclopent-2-en-1-yl acetate, 1-cyclohexylethyl (E/Z)-but-2-enoate, 1,5-dimethyl bicyclo[3.2.1]octan-8-one oxime, 2,4,6-trimethyl-4-phenyl-1,3-dioxane, 2-methyl-4-propyl-1,3-oxathiane, isopentyl acetate, ethyl 2-(2,4-dimethyl-1,3-dioxolan-2-yl)acetate, ethyl 2-methylbutanoate, (2-cyclopentylcyclopentyl)-(E/Z)-but-2-enoate, 4-methoxy-2-methyl-butane-2-thiol, 1,3-dimethylbutyl-(E/Z)-but-2-enoate, 1,3-dimethylbut-3-enyl-2-methylpropanoate, 1-methoxyhexane-3-thiol, 4-isopropyl-1-methyl-7-thiabicyclo[2.2.1]heptane, 2-(4-methylcyclohex-3-en-1-yl)propan-2-thiol, 5-methyl-2-(1-methyl-1-sulfanyl-ethyl)cyclohexanone, (1,3-dimethyl-3-phenyl-butyl)acetate, allyl 2-isopentyloxyacetate, 5-butyltetrahydrofuran-2-one, 5-pentyltetrahydrofuran-2-one, 5-heptyltetrahydrofuran-2-one, 5-octyltetrahydrofuran-2-one, 5-hexyltetrahydrofuran-2-one, ethyl 2-(2-methyl-1,3-dioxolan-2-yl)acetate and diethylcyclohexan-1,4-dicarboxylate,
and/or
the, one, several or all of the fragrance substance(s) of component (c), if present, is or are selected from the group consisting of 1-(2,6,6-trimethylcyclohex-2-en-1-yl)hepta-1,6-dien-3-one, 1-(2,6,6-trimethylcyclohex-2-en-1-yl)but-2-en-1-one, 1-(2,6,6-trimethylcyclohexen-1-yl)but-2-en-1-one, 1-(2,6,6-trimethylcyclohex-3-en-1-yl)but-2-en-1-on, 1-(2,4,4-trimethylcyclohex-2-en-1-yl)but-2-en-1-on, 1-methyl-4-(2,2,3-trimethylcyclopent-3-en-1-yl)-2-oxabicyclo[2.2.2]octane, 4-(4-methoxyphenyl)-butan-2-one, 5-methylhept-2-en-4-one, 6,6-dimethoxy-2,5,5-trimethylhex-2-ene and 2,4,6-trimethyl-4-phenyl-1,3-dioxane.

6. Method for producing a fragrance substance mixture, preferably a perfume oil, according to any of the claims 1 to 5, comprising or consisting of the following step:
- mixing component (a) as defined in any of the claims 1 to 5 with component (b) and/or (c) as defined in any of the claims 1 to 5.

7. Method according to claim 6, resulting in a fragrance substance mixture, preferably a perfume oil, in which
- the weight ratio of the total amount of fragrance substance(s) of component (b), if present, to the compound of formula (I) is greater than or equal to 40:60, preferably greater than or equal to 80:20, more preferably greater than or equal to 99:1,
and/or
- the weight ratio of the total amount of fragrance substance(s) of component (c), if present, to the compound of formula (I) is greater than or equal to 60:40, preferably greater than or equal to 80:20, more preferably greater than or equal to 90:10.

8. Method for enhancing the natural freshness and/or radiance and/or for masking or reducing sulphurous, artificial and/or metallic notes of one or more fragrance substances different from the compound of formula (I), preferably of one or more fragrance substances different from the compound of formula (I) having a fruity olfactory note, comprising the following step:
- mixing the fragrance substance(s) different from the compound of formula (I) with an amount of compound of formula (I) sufficient to enhance the natural freshness and/or radiance of the fragrance substance(s) different from the compound of formula (I) and/or to mask or reduce sulphurous, artificial and/or metallic notes of the fragrance substance(s) different from the compound of formula (I).

9. Method according to claim 8, wherein the, one, several or all of the fragrance substance(s) different from the compound of formula (I) is or are selected from the components (b) and/or (c) of a fragrance substance mixture according to any of the claims 1 to 5.

10. Method according to claim 9, wherein
- the weight ratio of the total amount of fragrance substance(s) of component (b), if present, to the compound of formula (I) is greater than or equal to 40:60, preferably greater than or equal to 80:20, more preferably greater than or equal to 99:1,
and/or
- the weight ratio of the total amount of fragrance substance(s) of component (c), if present, to the compound of formula (I) is greater than or equal to 60:40, preferably greater than or equal to 80:20, more preferably greater than or equal to 90:10.

11. Perfumed product containing a fragrance substance mixture, preferably a perfume oil, according to any of the claims 1 to 5, preferably in a sensorially effective amount.

12. Perfumed product according to claim 11, wherein the product is selected from the group consisting of perfume extracts, eau de parfums, eau de toilettes, aftershaves, eau de colognes, pre-shave products, splash colognes, perfumed refreshing wipes, acidic, alkaline and neutral detergents, textile fresheners, ironing aids, liquid detergents, powder detergents, laundry pretreatment agents, fabric softeners, washing soaps, washing tablets, disinfectants, surface disinfectants, air fresheners, aerosol sprays, waxes and polishes, personal care products, hand creams and lotions, foot creams and lotions, depilatory creams and lotions, aftershave creams and lotions, tanning creams and lotions, hair care products, deodorants and antiperspirants, decorative cosmetic products, candles, lamp oils, incense sticks, insecticides, repellents and propellants
and/or
wherein the proportion of the fragrance substance mixture according to any of the claims 1 to 5 in the perfumed product is 0.01 to 10% by weight, preferably 0.1 to 5% by weight, particularly preferably 0.25 to 3% by weight, based on the total weight of the perfumed product, respectively.

13. Method for producing a perfumed product, preferably a product according to claim 11 or 12, comprising or consisting of the following steps:
i) providing a fragrance substance mixture according to any of the claims 1 to 5 or producing a fragrance substance mixture by a method according to any of the claims 6 or 7,
ii) providing the further components of the perfumed product, and
iii) contacting the further components of the perfumed product provided in step ii) with a sensorially effective amount of the fragrance substance mixture provided in step i), preferably wherein the amount of the compound of formula (I) is sufficient to enhance the natural freshness and/or radiance of the, one, several or all fragrance substances of components (b) and/or (c) and/or to mask or reduce sulphurous, artificial and/or metallic notes of the, one, several or all fragrance substances of components (b) and/or (c),
or
I) providing the components of the perfumed product which are not components (a), (b) or (c) of a fragrance substance mixture according to any of the claims 1 to 5,
II) mixing the components of the perfumed product provided in step I) with the components (b) and/or (c) of a fragrance substance mixture according to any of the claims 1 to 5, so that a mixture results in which the total amount of the components (b) and/or (c) is present in a sensorially effective amount,
III) contacting the mixture prepared in step II) with an amount of the compound of formula (I), preferably wherein the amount of the compound of formula (I) is sufficient to enhance the natural freshness and/or radiance of the, one, several or all fragrance substances of the components (b) and/or (c) and/or to mask or reduce sulphurous, artificial and/or metallic notes of the, one, several or all fragrance substances of the components (b) and/or (c).

14. Use of the compound of formula (I) 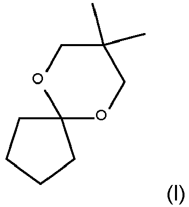 for enhancing the natural freshness and/or radiance and/or for masking or reducing sulphurous, artificial and/or metallic notes of one or more fragrance substances different from the compound of formula (I), preferably of fragrance substances having a fruity olfactory note,
wherein preferably the, one, several or all of the fragrance substances different from the compound of formula (I) is or are selected from the components (b) and/or (c) of a fragrance substance mixture according to any of the claims 1 to 5.

## Revendications

1. Mélange de substances odoriférantes, de préférence huile de parfum, comprenant ou se composant des composants suivants:
(a) le composé de formule (I) 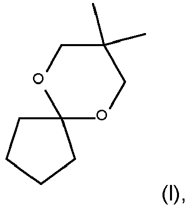 ainsi que, en sus,
(b) une ou plusieurs substance(s) odoriférante(s), de préférence ayant une note olfactive fruitée, choisie(s) dans le groupe constitué par les esters, les lactones, les oximes et les composés de soufre d'une masse molaire de 240 g/mol ou moins
et/ou
(c) une ou plusieurs substance(s) odoriférante(s) choisie(s) dans le groupe constitué par les acétals, les cétones et les éthers, ayant une masse molaire dans la plage allant de 126 à 240 g/mol.

2. Mélange de substances odoriférantes selon la revendication 1, dans lequel le composant (b) comprend deux, trois, quatre, cinq substances odoriférantes différentes ou plus
et/ou
le composant (c) comprend deux, trois, quatre, cinq substances odoriférantes différentes ou plus.

3. Mélange de substances odoriférantes selon la revendication 1 ou 2, dans lequel le rapport massique de la quantité totale de substance(s) odoriférante(s) du composant (b), s'il est présent, au composé de formule (I) est supérieur ou égal à 40 : 60, de préférence supérieur ou égal à 80 : 20, de manière particulièrement préférée supérieur ou égal à 99 : 1
et/ou
le rapport massique de la quantité totale de substance(s) odoriférante(s) du composant (c), s'il est présent, au composé de formule (I) est supérieur ou égal à 60 : 40, de préférence supérieur ou égal à 80 : 20, de manière particulièrement préférée supérieur ou égal à 90 : 10.

4. Mélange de substances odoriférantes selon l'une quelconque des revendications précédentes, dans lequel la, une, plusieurs ou toutes le(s) substance(s) odoriférante(s) du composant (b), s'il est présent, présente(nt) une masse molaire comprise entre 130 et 240 g/mol.

5. Mélange de substances odoriférantes selon l'une quelconque des revendications précédentes, dans lequel la, une, plusieurs ou toutes le(s) substance(s) odoriférante(s) du composant (b), s'il est présent, est ou bien sont choisie(s) dans le groupe constitué par le caproate d'allyle, le propionate d'allyle cyclohexyle, le 5-hexyl-4-méthyl tétrahydrofuran-2-one, le 2-cyclopent-2-én-1-ylacétate d'éthyle, le 1-cyclohexyléthyl-(E/Z)-but-2-énoate, le 1,5-diméthylbicyclo[3.2.1]octane-8-one oxime, le 2,4,6-triméthyl-4-phényl-1,3-dioxane, le 2-méthyl-4-propyl-1,3-oxathiane, l'acétate d'isopentyle, le 2-(2,4-diméthyl-1,3-dioxolan-2-yl)acétate d'éthyle, le 2-méthylbutanoate d'éthyle, le (2-cyclopentylcyclopentyl)-(E/Z)-but-2-énoate, le 4-méthoxy-2-méthyl-butan-2-thiol, le 1,3-diméthylbutyl(E/Z)-but-2-énoate, le 1,3-diméthylbut-3-ényl-2-méthylpropanoate, le 1-méthoxyhexane-3-thiol, le 4-isopropyl-1-méthyl-7-thiabicyclo[2.2.1]heptane, le 2-(4-méthylcyclohex-3-én-1-yl)propane-2-thiol, le 5-méthyl-2-(1-méthyl-1-sulfanyl-éthyl)cyclohexanone, le (1,3-diméthyl-3-phényl-butyl)acétate, l'allyl-2-isopentyloxyacétate, le 5-butyltétrahydrofurane-2-one, le 5-pentyltétrahydrofurane-2-one, le 5-heptyltétrahydrofuran-2-one, le 5-octyltétrahydrofurane-2-one, le 5-hexyltétrahydrofuran-2-one, le 2-(2-méthyl-1,3-dioxolan-2-yl)acétate d'éthyle et le diéthylcyclohexane-1,4-dicarboxylate,
et/ou
la, une, plusieurs ou toutes le(s) substance(s) odoriférante(s) du composant (c), s'il est présent, est ou bien sont choisie(s) dans le groupe constitué par le 1-(2,6,6-triméthylcyclohex-2-én-1-yle)hepta-1,6-dién-3-one, le 1-(2,6,6-triméthylcyclohex-2-én-1-yl)but-2-én-1-one, le 1-(2,6,6-triméthylcyclohexène-1-yl)but-2-én-1-one, le 1-(2,6,6-triméthylcyclohex-3-én-1-yl)but-2-én-1-one, le 1-(2,4,4-triméthylcyclohex-2-én-1-yl)but-2-én-1-one, le 1-méthyl-4-(2,2,3-triméthylcyclopent-3-én-1-yl)-2-oxabicyclo[2.2.2]octane, le 4-(4-méthoxyphényl)butan-2-one, le 5-méthylhept-2-én-4-one, le 6,6-diméthoxy-2,5,5-triméthyl-hex-2-en et le 2,4,6-triméthyl-4-phényl-1,3-dioxane.

6. Procédé de production d'un mélange de substance(s) odoriférante(s), de préférence d'une huile de parfum, selon l'une quelconque des revendications 1 à 5, comprenant ou se composant de l'étape suivante:
- mélanger le composant (a), tel que défini dans l'une quelconque des revendications 1 à 5, avec le composant (b) et/ou (c) tel(s) que défini(s) dans l'une quelconque des revendications 1 à 5.

7. Procédé selon la revendication 6, dans lequel un mélange de substances odoriférantes, de préférence une huile de parfum, résulte, dans lequel
- le rapport massique de la quantité totale de substance(s) odoriférante(s) du composant (b), s'il est présent, au composé de formule (I) est supérieur ou égal à 40 : 60, de préférence supérieur ou égal à 80 : 20, de manière particulièrement préférée supérieur ou égal à 99 : 1
et/ou
- le rapport massique de la quantité totale de substance(s) odoriférante(s) du composant (c), s'il est présent, au composé de formule (I) est supérieur ou égal à 60 : 40, de préférence supérieur ou égal à 80 : 20, de manière particulièrement préférée supérieur ou égal à 90 : 10.

8. Procédé destiné à intensifier la fraîcheur et/ou le rayonnement naturel(le)(s) et/ou à masquer ou réduire les notes sulfureuse, artificielle et/ou métallique d'une ou de plusieurs substance(s) odoriférante(s) différente(s) du composé de formule (I), de préférence d'une ou de plusieurs substance(s) odoriférante(s) différente(s) du composé de formule (I), ayant une note olfactive fruitée, comprenant l'étape suivante:
- mélanger la ou les substance(s) odoriférante(s) différente(s) du composé de formule (I) avec une quantité du composé de formule (I) qui est suffisante pour intensifier la fraîcheur et/ou le rayonnement naturel(le)(s) de la ou des substance(s) odoriférante(s) différente(s) du composé de formule (I) et/ou pour masquer ou réduire les notes sulfureuse, artificielle et/ou métallique de la ou des substance(s) odoriférante(s) différente(s) du composé de formule (I).

9. Procédé selon la revendication 8, dans lequel la, une, plusieurs ou toutes les substance(s) odoriférante(s) différente(s) du composé de formule (I) est ou bien sont choisie(s) parmi les composants (b) et/ou (c) d'un mélange de substances odoriférantes selon l'une quelconque des revendications 1 à 5.

10. Procédé selon la revendication 9, dans lequel:
- le rapport massique de la quantité totale de substance(s) odoriférante(s) du composant (b), s'il est présent, au composé de formule (I) est supérieur ou égal à 40 : 60, de préférence supérieur ou égal à 80 : 20, de manière particulièrement préférée supérieur ou égal à 99 : 1
et/ou
- le rapport massique de la quantité totale de substance(s) odoriférante(s) du composant (c), s'il est présent, au composé de formule (I) est supérieur ou égal à 60 : 40, de préférence supérieur ou égal à 80 : 20, de manière particulièrement préférée supérieur ou égal à 90 : 10.

11. Produit parfumé contenant un mélange de substances odoriférantes, de préférence une huile de parfum, selon l'une quelconque des revendications 1 à 5, de préférence en une quantité sensoriellement efficace.

12. Produit parfumé selon la revendication 11, dans lequel ledit produit est choisi dans le groupe constitué par les extraits de parfum, les eaux de parfum, les eaux de toilette, les eaux après-rasage, les Eaux de Cologne, les produits de pré-rasage, les Splash Cologne, les lingettes rafraîchissantes parfumées, les agents de nettoyage acides, alcalins et neutres, les produits à rafraîchir les textiles, les aides au repassage, les détergents liquides, les détergents en poudre, les produits de prétraitement de linge, les adousissants de linge, les savons à laver, les pastilles lave-linge, les désinfectants, les désinfectants de surface, les assainisseurs d'air, les aérosols, les cires et polis, les produits de toilette, les crèmes et lotions pour les mains, les crèmes et lotions pour les pieds, les crèmes et lotions dépilatoires, les crèmes et lotions après-rasage, les crèmes et lotions de bronzage, les produits de soins des cheveux, les désodorisants et antiperspirants, les produits de la cosmétique décorative, les bougies, les huiles de lampe, les bâtonnets d'encens, les insecticides, les répulsifs et les carburants
et/ou
dans lequel la proportion du mélange de substances odoriférantes selon l'une quelconque des revendications 1 à 5 dans le produit parfumé est comprise entre 0,01 et 10 % en poids, de préférence entre 0,1 et 5 % en poids, de manière particulièrement préférée entre 0,25 et 3 % en poids, respectivement par rapport à la masse totale du produit parfumé.

13. Procédé de production d'un produit parfumé, de préférence d'un produit selon la revendication 11 ou 12, comprenant ou consistant en les étapes suivantes:
i) fournir un mélange de substances odoriférantes selon l'une quelconque des revendications 1 à 5 ou produire un mélange de substances odoriférantes selon un procédé selon l'une quelconque des revendications 6 ou 7,
ii) fournir les autres composants du produit parfumé, et
iii) mettre en contact les autres composants du produit parfumé, fournis à l'étape ii), avec une quantité sensoriellement efficace du mélange de substances odoriférantes fourni à l'étape i), dans lequel, de préférence, la quantité du composé de formule (I) est suffisante pour intensifier la fraîcheur et/ou le rayonnement naturel(le)(s) de la, d'une, de plusieurs ou de toutes les substance(s) odoriférante(s) des composants (b) et/ou (c) et/ou pour masquer ou réduire les notes sulfureuse, artificielle et/ou métallique de la, d'une, de plusieurs ou de toutes les substance(s) odoriférante(s) des composants (b) et/ou (c),
ou
I) fournir les composants du produit parfumé qui ne sont pas de composants (a), (b) ou (c) d'un mélange de substances odoriférantes selon l'une quelconque des revendications 1 à 5,
II) mélanger les composants du produit parfumé, fournis à l'étape I), avec les composants (b) et/ou (c) d'un mélange de substances odoriférantes selon l'une quelconque des revendications 1 à 5, de sorte qu'il en résulte un mélange dans lequel la quantité totale des composants (b) et/ou (c) sont présents en une quantité sensoriellement efficace,
III) mettre en contact le mélange produit à l'étape II), avec une quantité du composé de formule (I), dans lequel, de préférence, la quantité du composé de formule (I) est suffisante pour intensifier la fraîcheur et/ou le rayonnement naturel(le)(s) de la, d'une, de plusieurs ou de toutes les substance(s) odoriférante(s) des composants (b) et/ou (c) et/ou pour masquer ou réduire les notes sulfureuse, artificielle et/ou métallique de la, d'une, de plusieurs ou de toutes les substance(s) odoriférante(s) des composants (b) et/ou (c).

14. Utilisation du composé de formule (I), 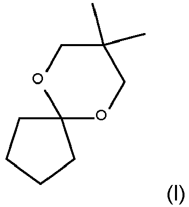
- pour intensifier la fraîcheur et/ou le rayonnement naturel(le)(s) et/ou pour masquer ou réduire les notes sulfureuse, artificielle et/ou métallique d'une ou de plusieurs substance(s) odoriférante(s) différente(s) d'une ou de plusieurs substance(s) odoriférante(s) différente(s) du composé de formule (I), de préférence de substances odoriférantes ayant une note olfactive fruitée,
dans lequel de préférence la, une, plusieurs ou toutes les substance(s) odoriférante(s) différente(s) du composé de formule (I) est ou bien sont choisie(s) parmi les composants (b) et/ou (c) d'un mélange de substances odoriférantes selon l'une quelconque des revendications 1 à 5.
